# EUROPEAN PATENT SPECIFICATION

(11) **EP 0 359 257 B1**
(45) Date of publication and mention of the grant of the patent: **12.04.1995**
(21) Application number: 89117029.2
(22) Date of filing: 14.09.1989
(51) Int. Cl.: A61K 31/66

(54) **Use of inositol triphosphate in the preparation of a medicament against diabetes**
Verwendung von Inositoltriphosphat zur Herstellung eines Arzneimittels gegen Diabetes
Utilisation du triphosphate d'inositol pour préparer un médicament contre le diabète

(30) Priority: 15.09.1988 SE 8803248
(43) Date of publication of application: 21.03.1990
(73) Proprietor: PERSTORP AB, 284 80 Perstorp (SE)
(72) Inventor: Sirén, Matti, CH-6926 Montagnola Lugano (CH)
(74) Representative: Hansen, Bernd, Dr. Dipl.-Chem.

(56) References cited:
- EP-A- 0 179 439
- EP-A- 0 179 440
- THE JOURNAL OF NEUROSCIENCE, vol. 8, no. 7, July 1988, pages 2544-2555, Society for Neuroscience; L.A. FINK et al.: "Inositol trisphosphate releases intracellularly stored calcium and modulates ion channels in molluscan neurons"
- LANCET, vol. 2, no. 8103, December 1978, pages 1282-1284; J.G. SALWAY et al.: "Effect of myo-inositol on peripheral-nerve function in diabetes"
- DIABETES, vol. 37, no. 11, November 1988, pages 1542-1548; H. XIANG et al.: "Effect of myo-inositol and T3 on myocardial lipids and cardiac function in streptozocin-induced diabetic rats"

## Description

The present invention relates to the use of at least one isomer of inositoltrisphosphate (IP₃) for the preparing of a medicament for preventing, alleviating or combatting complications of diabetes in mammals including man. The invention also relates to a medicament comprising at least one isomer of IP₃ and an anti-diabetic compound effective against diabetes and complications thereof in mammals including man.

Diabetes can be divided in two main types i.e. Insipidus and Mellitus. The former type is caused by underproduction of pituitrin, an anti-diuretic hormone produced by the pituitary gland.
The latter type is further divided in two main types: type I, Insulin Dependent Diabetes Mellitus (IDDM) or type II, Non Insulin Dependent Diabetes Mellitus (NIDDM). Insulin deficiency caused by destruction of the beta-cells of the islets of Langerhans is the major characteristic of type I diabetes, while the second type is thought to be caused by defects in insulin receptors or diminished secretion of insulin. Most of the diabetics belong to type II and are often over forty and obese.
Symptoms of the disease may include excessive urination thirst and hunger, vision can detoriate and foot ulceration and poorly healing skin wounds often occur. Furthermore patients may display neuritis, ketosis, hyperglycaemia, glycosuria and in some cases coma or ketoacidosis.

The focus of the treatment of diabetes per se is to regulate and control the levels of glucose in the body.

However complications of diabetes occur due to undiagnosed and untreated disease or poorly controlled medication of the disease. Essentially this means that the problems and treatments of diabetes and complications thereof are quite separate.

Short-term complications are ketoacidosis or coma and infections.
Absence or inadequate amounts of insulin causes a changed metabolism of carbohydrates, fat and protein. The number of ketone bodies are increased in these cases and followed by manifestations of ketoacidosis such as polyuria, thirst and general ill-feeling. These conditions can eventually lead to coma.
Infections in diabetics are serious due to a decreased resistance caused by hyperglycaemia. Examples of infections are infections of the urinary tract and fungal infections.
Long term complications are neuropathy, retinopathy and vascular complications.
Peripheral nerves are most frequently involved in neuropathy. Symptoms could be pain, absence of reflexes and muscle weakness.

Thickening of the capillary-basement membranes of many organs causes vascular complications such as microangiopathy. Renal diseases such as nephropathy characterized by changes in the glomeruli is one example while others are skin diseases and an increased prevalence of arteriosclerosis which can result in strokes, myocardial infarction, intermittent claudication and gangrene.
Various types of ocular complications occur but retinopathy is the most prevalent. Impairment of retinal circulation causes haemorrhage and loss of vision.

Diabetes type I is treated with insulin while type II is treated with hyperglycaemic agents such as sulphonylureas and biguanides.
Existing drugs often suffer from limited effectiveness in combination with serious side effects. Insulin therapy is burdensome for the patient as the drug has to be injected many times a day. Furthermore allergic reactions towards insulin often appears. Insulin dosages have to be very carefully controlled as an excess could lead to hypoglycaemia. Fluctuations in blood glucose due to wrong diet and/or badly controlled insulin dosage are very negative for the patient.
Sulphonylureas causes side-effects such as gastrointestinal disturbances, hypersensitivity reactions, hypoglycaemia and an increased risk for cardiovascular diseases.
Biquanides could cause side-effects such as anorexia, vomiting and diarrhoea.
Furthermore the existing therapies used for treatment of diabetes do not arrest the complications of diabetes to any significant extent. Since some years there is an effort to develop drugs against complications of diabetes. So called aldose reductase inhibitors have shown certain effects against these diseases but only at high dosage and with serious side effects. This implies a marked need for more effective and safe pharmacological compounds in this area.

According to the present invention it has surprisingly become possible to overcome and reduce the above mentioned complications of diabetes by the use of at least one isomer of inositoltrisphosphate (IP₃) for the preparing of a medicament for preventing alleviating or combatting complications of diabetes in mammals including man, wherein the complications are selected to cataract formation, neuropathy, nephropathy, retinopathy, vascular complications or wounds. In additon the present invention relates to an anti-diabetic pharmaceutical composition comprising at least one isomer of IP₃ in combination with a pharmaceutically active anti-diabetic compound. The invention also covers the use of at least one isomer of IP₃ in combination with a pharmaceutically active anti-diabetic compound for preparing of a medicament for preventing, alleviating or combatting diabetes or complications thereof.

From the European Patent No 179439 a pharmaceutical composition comprising as a pharmaceutically active ingredient at least one isomer of inositoltrisphosphate is known. In said patent the effect of this pharmaceutical composition is shown for different areas. Among others the effect of the compositon for reduction of blood glucose is shown. No mention is made however of a possibility to prevent, alleviate or combat complications of diabetes. As is wellknown in this field antidiabetic agents which reduce blood glucose levels in many cases fail to prevent or even alleviate complications of diabetes as described hereinbefore.
The present invention relates to the use of IP₃ for preparing a medicament for preventing, alleviating or combatting short-term and long-term complications of diabetes such as:
Neuropathy manifestated for example by numbness, tingling, pain, loss of reflexes and muscle weakness. The disease is classified as symmetric distal polyneuropathy, asymmetric neuropathy and autonomic neuropathy. Often neuropathic lesions result in ulcers for example in the foot;
Vascular complications characterized by thickening of the capillary basement membrane in many organs i.e. microangiopathy. Examples of these diseases are renal diseases related to changes in the glomeruli such as glomerulopathy, pyelonephritis, necrosis and nephropathy. Microangiopathy of the vessels supplying the skin, peripheral nerves and walls of arteries causes skin diseases, arteriosclerosis, stroke, myocardial infarction and intermittent claudication;
Complications affecting vision such as retinopathy, nonproliferative or proliferative, changes in refarction, glaucoma and cataracts;
Metabolic complications such as ketoacidosis, lactic acidosis, coma and hypoglycemia. Ketoacidosis is characterized by decreased metabolism of carbohydrates and increased breakdown of fat and protein resulting in increased amounts of ketone bodies;
Infections; especially in the urinary tract, fungal infections and susceptibility to tuberculosis increases as the resistance is decreased.

In preferred embodiments of the invention the complications of diabetes relates to vascular complications, nephropathy, neuropathy and retinopathy.

The production of IP₃ and the isolation of the different isomers thereof are disclosed in the US patent No 4.777.134. The IP₃ isomers can also be produced by synthetic methods, chemically or enzymatically, starting with e.g. inositol and a phosphorus source. Furthermore, microbiological production methods including hybrid DNA-techniques of IP₃ are also suitable.

The structure of IP₃ and the different isomers thereof are disclosed in the US patent No 4.735.936 and the US patent No 4.797.390.

It is suitable that the medicament used according to the invention exists in unit dosage form. Tablets, granules or capsules are suitable administration forms for such unit dosage. Furthermore, tablets and granules can easily be surface treated such as to provide an enteric coating to prevent an uncontrolled hydrolysis in the stomach and to bring about a desired absorption in the intestine. Other suitable administration forms are slow release and transdermal administration. A usual pharmaceutically acceptable additive, excipient and/or carrier can be included in the medicament. The tablets or granules can also contain a disintegrant which causes the tablets or the granules, respectively, to disintegrate easily in the intestine. In certain cases, especially in acute situations, it is preferable to use the unit dosage in the form of a solution for intravenous administration.

The medicament can also consist as such of IP₃ solely without any additive, excipient or carrier.

If desired, the medicament can be free of other inositol phosphates, IP₁, IP₂, IP₄, IP₅ and IP₆. Accordingly, the mixture of IP₃ isomers can have a purity of 90-100%, such as 93-100% or preferably 95-100%.

Alternatively, the medicament can consist of or comprise one or more specific IP₃ isomers, each present in substantially pure form. Thus, the different isomers can be isolated from each other in substantially pure form, which means that they have a purity of 80-100%, such as 82-100% or 85-100%, preferably 90-100%. Since the isomers can be produced in pure form they can be mixed in any proportion, of course.

The medicament can consist of IP₃, wherein said IP₃ is provided by at least one of IP₆, IP₅ or IP₄ and a degradative substance such as an enzyme suitable to form IP₃.

It is in most cases suitable that the IP₃-isomer or isomers used for the preparing of the medicament according to the invention is present in salt form in order not to affect the mineral balance negatively. The salt should preferably consist of a sodium, calcium, zinc or magnesium salt or a mixture of two or more of these salts. Calcium and zinc salts or mixtures of these are especially preferred. The isomer of IP₃ can also partly be present as a salt of one or more physiologically acceptable compounds in the lanthanide series.

For the above mentioned reasons it is also an advantage if the medicament contains a surplus or an extra addition of at least one pharmaceutically acceptable salt of calcium, zinc or magnesium with a mineral acid or organic acid. This is especially valuable for elderly persons who are often deficient in these minerals.

For administration to human patients appropriate dosages can routinely be determined by those skilled in this art by extension of the results obtained in animals at various dosages. The preferred dosage for humans falls within the range of 0.1 to 1000 mg, especially 0.1 to 200 mg IP₃/day/kg body weight.

In animal experiments, no toxic effects were seen after administration of very high doses of IP₃, 160 mg/kg body weight by intraperitoneal injection to mice.

The medicament usually contains 0.01-1.5 g, such as 0.05-1.3 or preferably 0.1-1 g of IP₃ per unit dosage.

In one preferred embodiment of the invention the IP₃ is D-myo-inositol-1.2.6-trisphosphate.

One type of function of the medicament is to reverse, prevent or alleviate damage to membranes of different cell types, but especially cell membranes of platelets, erythrocytes and endothelial cells. The use of the medicament results in an improved stabilization, a decreased susceptibility to deformation and an improved function of the cell types. Furthermore, parameters such as aggregability and adhesion to e.g. vessel walls are decreased.
When the medicament is used extravasation is decreased and a reduction of vascular leakage and blood flow is observed.

The release of mediators such as serotonin from platelets are normalized when the medicament is used. Furthermore a normalization of the inositol metabolism is measured when the medicament is used. Other results of the use of the medicament are regulation of membrane fluidity, the incorporation of components such as cholesterol and the production, incorporation and balance between different phospholipids.

Another result of the use of the medicament is the regulation of the electrolyte balance of the cell. As examples, regulation of intracellular levels of calcium, potassium, chloride, phosphate etc. can be mentioned.

The cell surface activity and responsiveness to external stimuli via receptor activation /deactivation due to for example phosphorylation are other parameters effected by the use of the medicament.

As mentioned above the invention also relates to an anti-diabetic pharmaceutical composition comprising at least one specific isomer of IP₃ in combination with a pharmaceutically active anti-diabetic agent. Such a composition allows a reduction in the dosage of the anti-diabetic compound, which reduces the above mentioned side effects. Furthermore an improved profile of action is obtained. The pharmaceutical composition could be in a combined dosage form or in a separate dosage form for separate or combined administration.

The anti-diabetic compound can be insulin of different origins or derivatives and analogues thereof such as proinsulin or a hypoglycaemic agent selected from the groups of sulphonylureas such as tolbutamide, chlorpropamide, acetohexamol, glibenclamide, glipizide, gliclazide, glybornuride, acetohexamide and tolazamide or biguanides such as phenformin and metformin. Furthermore the composition can comprise in combination with IP₃ cyclosporin, ciamexone, glucosidase inhibitors, amylase inhibitors and mediators of growth hormones such as somatomedins.
The amount of IP₃ should constitute 5 to 95 or 15 to 80 such as 25 to 60 per cent by weight of said active ingredients. In one preferred embodiment of the invention, relating to the combined composition, the IP₃ is D-myo-inositol-1.2.6-trisphosphate.

The medicament, prepared from the combination, could be used for example for preventing, alleviating or combatting the following conditions:
Diabetes insipidus and mellitus including type I (IDDM) and type II (NIDDM), secondary diabetes, impaired glucose tolerance and gestational diabetes which all lead to hyperglycemia;
Complications of diabetes such as neuropathy, vascular complications e.g. microangiopathy and nephropathy, retinopathy, ketoacidosis and increased susceptibility to infections.

The invention will be further explained with the following examples. Examples 1-4 show the production of IP₃ whereas examples 5 and 6 illustrate the preparation of solutions and tablets of IP₃. Example 7 relates to the effect of IP₃ on diabetes-induced platelet aggregation while examples 8-11 describe the beneficial effect of IP₃ on complications of diabetes. In example 12 decreased microbleeding in the presence of IP₃ is shown while example 13 illustrates the impact of IP₃ on a specific enzyme, an ATPase.

### Example 1

### Hydrolysis of sodium phytate with baker's yeast and fractionation of a mixture of inositol phosphates.

A 1.4 kg quantity of sodium phytate (from corn, Sigma Chemical Co) was dissolved in 1 200 l sodium acetate buffer pH 4.6. 100 kg of baker's yeast from Jästbolaget, Sweden (dry substance: 28 %, nitrogen content: 2 %, phosphorus content: 0.4 %) was added with stirring and incubation was continued at 45°C. The dephosphorylation was followed by determining the inorganic phosphorus released. After 7 hours when 50 % inorganic phosphorus was liberated the hydrolysis was stopped by adding ammonia to pH 12. The suspension was centrifuged and the supernatant was collected.

800 l of the supernatant was passed through an ion-exchange column (Dowex® 1, chloride form, 100 cm x 150 cm) and eluted with a linear gradient of hydrochloric acid (0-0.7 N HCL).

### Example 2

### Structural determination of isomers of inositol triphosphate.

The fraction obtained in example 1 with a phosphorus/ inositol ratio of three to one was neutralized and precipitated as a calciumsalt. 100 mg of the precipitate was analyzed with H-NMR. Data show that the peak consists of myo-inositol-1.2.6-trisphosphate.

### Example 3

A 0.5 gram quantity of D-chiro-inositol was dissolved in 1 ml phosphoric acid at 60°C. 20 g polyphosphoric acid was added and the mixture was heated to 150°C under vacuum for 6 hours. The mixture was diluted with water to a volume of 200 ml and passed through an ion-exchange column (Dowex® 1, chloride form, 25 mm x 250 mm) and eluted with a linear gradient of hydrochloric acid (0-2.0 N HCl).

The content of the peak with the ratio of phosphorus to inositol of six to one was precipitated by addition of calcium-hydroxide. The precipitate was filtered, washed and mixed with 10 ml of a cation-exchange resin to give the acid form of the inositolhexaphosphate. After neutralization with sodium hydroxide and freeze-drying the sodium salt of D-chiro-inositolhexaphosphate was obtained.

### Example 4

A 0.8 gram quantity of the sodium salt of D-chiro-inositolhexaphosphate produced according to Example 3 was dissolved in 300 ml sodium acetate buffer, pH 5.2. 1.3 gram wheat phytase (EC 3.1.3.26, 0.015 U/mg from Sigma Chemical Co.) was added and the mixture was incubated at 38°C.

After the liberation of 50 % inorganic phosphorus the hydrolysis was stopped by adding ammonia to pH 12.

The mixture containing D-chiro-inositolphosphates was passed through an ion-exchange column (Dowex 1 chloride form, 25 mm x 250 mm) and eluted with a linear gradient of hydrochloric acid (0-0.7 N HCL).

The peak with the ratio of phosphorus to inositol of three to one was neutralized with 1.0 M sodium hydroxide and freeze-dried.

Structural determination with NMR and IR showed the product to be D-chiro-inositoltriphosphate.

### Example 5

### Solution of sodium salt of D-myo-inositol-1.2.6-triphosphate for injection.

0.5 g of the sodium salt of IP₃ and 0.77 g NaCl were dissolved in 98.73 ml of water for injection to form a solution suitable for injection into a person or an animal.

### Example 6

### Tablets of calcium salt of D-myo-inositol-1.2.6-triphosphate.

Tablets of the calciun salt of D-myo-inositol-1.2.6-triphosphate were produced in the following way. 50 g calcium salt of D-myo-inositol-1.2.6-triphosphate, 132 g lactose and 6 g acacia were mixed. Purified water was then added to the mixture, whereupon the mixing was continued until a suitable consistency was obtained. The mixture was sieved and dried. Then the mixture was blended with 10 g talcum and 2 g magnesium stearate. The mixture was compressed into tablets each weighing 200 mg.

### Example 7

Two groups of male rats with an initial body weight ranging between 300 g and 250 g were used. They were fed a diet comprising casein (17 %), starch (39 %), butter and different oils such as sunflower oil (18 %) and glucose, fructose, vitamin etc. (26 %).

On the first day on the experiment streptozotocin (Sigma) was injected intraperitoneally in order to induce complications of diabetes in the animals.

One of the groups was given D-myo-inositol-1.2.6-trisphosphate (IP₃), 160 ppm, in the diet and the other group served as a control.

Platelet aggregation in response to thrombin was measured after two months.

The results are as follows:

| Treatment | Thrombin induced platelet aggregation (cm) | Number of animals |
|---|---|---|
| Control | 35.0 +/- 2.1 | 8 |
| IP₃ | 18.9 +/- 5.2 | 11 |

Thus, after two months of supplementation of IP₃ thrombin induced aggregation was significantly lower in the IP₃-treated animals compared to the controls.

### Example 8

Two groups of rats were treated as described in Example 7.

The formation of cataracts in the eyes of the animals was measured after four months.

| Treatment | Cataract formation (% of all eyes) | Number of animals |
|---|---|---|
| Control | 38.5 % | 8 |
| IP₃ | 12.5 % | 11 |

The formation of cataracts is a serious complication of diabetes. After four months of treatment with IP₃ the formation of cataracts was markedly decreased.

### Example 9

Two groups of rats were treated as described in Example 7. In a diabetic condition the metabolism of lipids is disturbed, which is a serious complication of diabetes. One measurement of this disturbance is the formation of so called ketone bodies found in the urine.

While the amount of ketone bodies in the control group was high, the treatment with IP₃ resulted in a total elimination of ketone bodies in the urine of this group.

### Example 10

Increased metabolism of glucose to sorbitol in diabetics is linked to increases in blood flow and increased permeation of albumin in many tissues, which result in complications of diabetes.
In a skin chamber model rats are exposed to glucose and increases in blood flow is assessed by 15»m ⁸⁵ Sr-microspheres while vascular leakage is determined by the distribution and permeation of ¹²⁵I-albumin.
Bilateral skin chambers were mounted on eight normal Sprague-Dawley male rats after removal of a 2 cm circle of skin. Starting 10 days later 0.5 ml of 30 mM D-glucose in saline was added to one chamber in four animals while one chamber in these four animals only was supplied with saline. In a parallell experiment four other animals received 0,5 ml of 30 mM D-glucose in saline in one of the chambers while the other chamber in these four animals were supplied with 0,3 mg/kg/day D-myo-inositol-1.2.6-trisphosphate (IP₃). The treatment continued for another 10 days. The animals were then anesthetized with Inactin and blood flow and permeation of albumin were assessed.
Blood flow was increased 50 % with the addition of D-glucose compared to control while the blood flow was normalized when also IP₃ was given together with D-glucose. The permeation of albumin was increased six times with the addition of D-glucose compared to control while this increased permeation was diminished with 50 % when IP₃ was given together with D-glucose. These results show that IP₃ significantly decreases blood flow and protein permeability i.e. reduces vascular leakage.

### Example 11

One serious type of complications of diabetes is neuropathy. The decrease of motor nerve conduction velocity (MNCV) is an important characteristic of the condition.
Three groups of rats were used in this example. The first group, n=12, served as a control without diabetes. The two other groups, n=14 and n=10, respectively were given streptozotocin (STZ) in order to induce diabetes. One of these latter groups did not get any further treatment while the other group was treated daily with D-myo-inositol-1.2.6-triphosphate (IP₃, 1g/kg diet) mixed in the diet for 10 weeks. At the end of this period the animals were killed and MNCV were assessed in homogenates of sciatic nerves. The results for the different groups are shown below:

| Treatment | MNCV (m/s) |
|---|---|
| Control | 61.9 |
| STZ | 48.3 |
| STZ and IP₃ | 54.1 |

The treatment with IP₃ thus counteracts the impaired nerve conduction velocity, which implies a beneficial effect on neuropathy.

### Example 12

Vascular permeability and bleeding are important factors of complications of diabetes. These factors were induced by histamine in the hamster cheek pouch and were determined in the presence of D-myo-inositol-1.2.6-trisphosphate (IP₃) compared to control.
Male hamsters were fasted overnight and anaesthetised with sodium pentobarbitone. Infusion was performed through the jugular vein. Injection of histamine (2.7 mg/kg) was preceded by either saline (control) or IP₃ (5.0 mg/kg). The cheek pouch was observed with a microscope for a period of 80 minutes and the number of bleeding sites (petechiae) was recorded before the commencement of the infusion of histamine and at the end of the 80 min period.
The results are shown in the following table:

| Treatment | number of animals | number of petechiae | % inhibition |
|---|---|---|---|
| Saline (control) | 10 | 8.4 | - |
| IP₃ | 6 | 1.0 | 88 |

It can be seen that IP₃ is an effective compound against increased vascular permeability and bleeding.

### Example 13

Fresh blood was centrifuged at 200 g for 20 mins to prepare platelet-rich plasma. This was removed and after acidification to pH 6.4 by addition of citric acid it was centrifuged for 20 mins. The platelet-pellet was resuspended in a buffer containing 0.34 M sorbitol and 10 mM Hepes, pH 7.4.
The platelet-suspension was sonicated and the homogenate was layered onto a 1.0-3.5 M linear sorbitol density gradient. A mixed membrane fraction was removed from the gradient and concentrated before measurements of ⁴⁵Ca-sequestration and ATPase activity.

To one part of the platelet preparation 100 »M D-myo-inositol 1.2.6-trisphosphate (IP₃) was added while the other part served as a control. The addition of IP₃ increases the uptake of Ca into the endoplasmatic reticulum by 30 % and stimulates significantly the activities of both Mg ATPase and Ca Mg ATPase in the preparation.
Decreased activity of ATPases is observed in different cell-types in diabetics and the results in the example show that IP₃ counteracts the decreased activity.

## Claims

1. The use of at least one isomer of inositoltriphosphate (IP₃) for the preparing of a medicament for preventing, alleviating or combatting complications of diabetes in mammals including man, wherein the complications are related to cataract formation, neuropathy, nephropathy, retinopathy, vascular complications or wounds.

2. The use of at least one isomer of inositoltriphosphate (IP₃) in combination with an anti-diabetic compound for preparing a medicament for combined or separate administration in the preventing, alleviating or combatting diabetes or complications thereof.

3. The use according to claim 2, wherein the said diabetes or complications thereof are related to hyperglycemia, hyperketonemia, cataract formation, retinopathy, nephropathy, neuropathy, vascular complications or wounds.

4. A pharmaceutical composition for preventing, alleviating or combatting complications of diabetes, wherein the said diabetes or complications thereof are related to hyperglycemia, hyperketonemia, cataract formation, retinopathy, nephropathy, neuropathy, vascular complications or wounds, comprising at least one specific isomer of inositoltriphosphate (IP₃) and an anti-diabetic compound in a separate or combined dosage form.

5. A composition according to claim 4, wherein the anti-diabetic compound is insulin or derivatives of insulin.

6. A composition according to claim 4, wherein the anti-diabetic compound is a hypoglycaemic agent.

7. A composition according to any one of claims 4 to 6, wherein the isomer of inositoltriphosphate is D-myo-inositol-1,2,6-triphosphate.

## Patentansprüche

1. Verwendung wenigstens eines Isomeren von Inositoltriphosphat (IP₃) zur Herstellung eines Medikaments zur Vorbeugung, Linderung oder Bekämpfung von Komplikationen durch Diabetes in Säugern einschließlich des Menschen, wobei die Komplikationen die Kataraktbildung, Neuropathie, Nephropathie, Retinopathie, vaskulare Komplikation oder Wunden betreffen.

2. Verwendung wenigstens eines Isomeren von Inositoltriphosphat (IP₃) in Kombination mit einer antidiabetischen Verbindung zur Herstellung eines Medikaments zur gemeinsamen oder getrennten Verabreichung bei der Vorbeugung, Linderung oder Bekämpfung von Diabetes oder damit verbundenen Komplikationen.

3. Verwendung gemäß Anspruch 2, wobei die Diabetes oder die damit verbundenen Komplikationen Hyperglykämie, Hyperketonämie, Kataraktbildung, Retinopathie, Nephropathie, Neuropathie, vaskulare Komplikationen oder Wunden betreffen.

4. Pharmazeutische Zubereitung zur Vorbeugung, Linderung oder Bekämpfung von Komplikationen durch Diabetes, wobei die Diabetes oder die damit verbundenen Komplikationen Hyperglykämie, Hyperketonämie, Kataraktbildung, Retinopathie, Nephropathie, Neuropathie, vaskulare Komplikationen oder Wunden betreffen, die wenigstens ein Isomer von Inositoltriphosphat (IP₃) und eine antidiabetische Verbindung in getrennter oder gemeinsamer Dosierungsform umfaßt.

5. Zubereitung gemäß Anspruch 4, in der die antidiabetische Verbindung Insulin oder Insulin-Derivate ist.

6. Zubereitung gemäß Anspruch 4, in der die antidiabetische Verbindung ein hypoglykämisches Mittel ist.

7. Zubereitung gemäß einem der Ansprüche 4 bis 6, der das Inositoltriphosphat-Isomer D-Myo-inositol-1,2,6-triphosphat ist.

## Revendications

1. Utilisation d'au moins un isomère du triphosphate d'inositol (IP₃) pour la préparation d'un médicament destiné à prévenir, soulager ou combattre les complications du diabète chez des mammifères, y compris l'homme, les complications étant liées à la formation d'une cataracte, à la neuropathie, à la néphropathie, à la rétinopathie, aux complications vasculaires ou aux plaies.

2. Utilisation d'au moins un isomère du triphosphate d'inositol (IP₃), en combinaison avec un composé anti-diabétique, pour la préparation d'un médicament destiné à prévenir, soulager ou combattre le diabète ou ses complications, en administration conjointe ou séparée.

3. Utilisation conforme à la revendication 2, dans laquelle ledit diabète ou ses complications sont liés à une hyperglycémie, une hypercétonémie, la formation d'une cataracte, la rétinopathie, la néphropathie, la neuropathie, les complications vasculaires ou les plaies.

4. Composition pharmaceutique destinée à prévenir, soulager ou combattre les complications du diabète, ledit diabète ou ses complications étant liés à une hyperglycémie, une hypercétonémie, la formation d'une cataracte, la rétinopathie, la néphropathie, la neuropathie, les complications vasculaires ou les plaies, ladite composition comprenant au moins un isomère particulier du triphosphate d'inositol (IP₃) et un composé antidiabétique, sous forme posologique séparée ou combinée.

5. Composition conforme à la revendication 4, dans laquelle le composé antidiabétique est de l'insuline ou des dérivés de l'insuline.

6. Composition conforme à la revendication 4, dans laquelle le composé antidiabétique est un agent hypoglycémiant.

7. Composition conforme à l'une des revendications 4 à 6, dans laquelle l'isomère du triphosphate d'inositol est le D-myo-inositol-1,2,6-triphosphate.
